Europäisches Patentamt

European Patent Office

Office européen des brevets

(11) Numéro de publication : **0 080 152**
**B1**

# ·FASCICULE DE BREVET EUROPÉEN

(45) Date de publication du fascicule du brevet :
12.11.86

(51) Int. Cl.⁴ : **A 41 B 13/02**

(21) Numéro de dépôt : **82110575.6**

(22) Date de dépôt : **16.11.82**

(54) Dispositif d'attache par adhésif pour une couche-culotte à jeter et couche-culotte à jeter équipée de tels dispositifs d'attache.

(30) Priorité : **25.11.81 FR 8122100**

(43) Date de publication de la demande :
**01.06.83 Bulletin 83/22**

(45) Mention de la délivrance du brevet :
**12.11.86 Bulletin 86/46**

(84) Etats contractants désignés :
**BE DE GB IT**

(56) Documents cités :
**FR-A- 2 259 553**
**FR-A- 2 272 614**
**US-A- 4 005 712**
**US-A- 4 014 339**
**US-A- 4 047 529**

(73) Titulaire : **BOUSSAC SAINT FRERES B.S.F. Société anonyme dite:**
**12, rue du Vieux Faubourg**
**F-59800 Lille (FR)**

(72) Inventeur : **Lucas, Gérard**
**2, avenue des Jonquilles**
**F-59166 Bousbecque (FR)**
Inventeur : **Dussaud, Jacques**
**26, avenue du Maréchal Leclerc**
**F-59110 La Madeleine (FR)**

(74) Mandataire : **Casalonga, Axel et al**
**BUREAU D.A. CASALONGA OFFICE JOSSE & PETIT**
**Morassistrasse 8**
**D-8000 München 5 (DE)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention se rapporte à une couche-culotte à jeter, notamment pour enfants en bas âge, comprenant une mince feuille extérieure imperméable, et un matelas absorbant, et, de préférence, une feuille interne perméable, avec des dispositifs d'attache réouvrables au nombre de deux servant à fixer l'une à l'autre les parties avant et arrière de la couche-culotte.

Des dispositifs d'attache par adhésif pour des couches-culottes sont bien connus (voir par exemple brevets US 3.180.355, 3.630.201, 4.047.530, 4.049.001, 4.050.453 et brevet français n° 74 36 169). Chaque dispositif d'attache comprend généralement une languette adhésive fixée sur une partie de la feuille extérieure de la couche-culotte, notamment de la partie arrière, et venant se coller, lors de la fermeture de la couche-culotte, sur l'autre partie de la feuille extérieure, notamment sur la partie avant.

Il arrive fréquemment que l'on veuille rouvrir la couche-culotte, par exemple pour vérifier si l'enfant a mouillé le matelas absorbant, et la refermer si le matelas absorbant n'est pas encore mouillé. Le même problème de réouverture et de refermeture de la couche-culotte peut se présenter lorsqu'on veut rectifier la tenue de la couche-culotte, par exemple en vue de la resserrer à la taille. Or, il s'avère qu'une telle tentative d'ouverture sur une couche-culotte à jeter sur laquelle l'enveloppe extérieure est constituée par une feuille mince provoque, soit la déchirure de la languette adhésive soit l'arrachement de la zone de la feuille à laquelle adhère la languette adhésive, cette zone arrachée de la feuille restant collée sur la languette adhésive. Dans un cas comme dans l'autre, il n'est plus possible de refermer la couche-culotte qui est ainsi inutilisable, bien que le matelas absorbant puisse ne pas encore être mouillé.

Pour permettre une telle réouverture et refermeture d'une couche-culotte à jeter, on a déjà proposé des dispositifs d'attache par adhésif (brevets US n° 3.989.048 et 4.014.339) dont les languettes adhésives en forme de double rabat replié en U ou en Z comprennent deux parties recouvertes d'une couche d'adhésif, ces deux parties pouvant être utilisées successivement en vue d'une première fermeture et d'une seconde fermeture de la couche-culotte. Toutefois, ces languettes à double fermeture sont de structure relativement compliquée puisqu'elles impliquent l'utilisation d'un rabat à pliage multiple, portant en plusieurs endroits bien définis des couches d'adhésif et des couches anti-adhésives. L'utilisation de ces dispositifs connus, notamment en vue de la seconde fermeture, ne présente pas non plus la simplicité requise pour ce genre d'articles et implique en particulier une déchirure du rabat en deux endroits bien déterminés.

On connaît par ailleurs un dispositif d'attache à utilisations multiples pour une couche-culotte à jeter (brevet US n° 4.034.752) comprenant plusieurs languettes adhésives individuelles qui sont toutes fixées de façon détachable sur une partie de la feuille extérieure recouverte d'une couche anti-adhésive ; pour chaque fermeture, on détache une languette et on la colle sur les deux parties de la feuille extérieure, devant être reliées entre elles.

Enfin, par les brevets US n° 3.999.546 et 4.049.001, on connaît des dispositifs d'attache par adhésif avec deux rabats ou languettes superposés. Ces dispositifs sont relativement épais et présentent surtout l'inconvénient que l'on risque, à la première fermeture, d'utiliser par mégarde le rabat ou la languette destiné à la seconde fermeture de sorte que le dispositif d'attache ne peut plus servir à une seconde fermeture.

La présente invention a pour objet une couche-culotte à jeter équipée de dispositifs d'attache par adhésif, permettant, après une première fermeture, une réouverture et une seconde fermeture de la couche-culotte, tout en étant d'une structure et d'une fabrication simples et d'une utilisation sûre et aisée.

Telle qu'elle est revendiquée, la couche-culotte à jeter de l'invention comprend une mince feuille extérieure imperméable, un matelas absorbant et de préférence une feuille interne perméable, avec deux dispositifs d'attache par adhésif placés sur les bords latéraux de la couche-culotte et destinés à fixer l'une à l'autre les parties avant et arrière de la couche-culotte. Chaque dispositif d'attache comprend : un rabat de fixation qui présente à une extrémité une première partie fixée de façon non détachable par une couche d'adhésif sur la face externe de la partie arrière de ladite feuille imperméable jusqu'au bord latéral de ladite partie arrière, ainsi que, à son autre extrémité, une seconde partie qui est destinée au cours de l'utilisation, à être fixée par une couche d'adhésif sensible à la pression sur la face externe de la partie avant de la feuille imperméable. Ledit rabat comprend, entre lesdites première et seconde parties, une troisième partie qui est munie de la même couche d'adhésif sensible à la pression que lesdites première et seconde parties et qui est recouverte d'un élément de masquage dont la face tournée vers ladite couche d'adhésif dudit rabat est munie d'une couche anti-adhésive. Selon l'invention, chaque dispositif d'attache comprend en outre un élément d'accrochage fixé de façon non détachable par une couche d'adhésif au voisinage du bord de la face interne de la partie arrière de ladite feuille imperméable et muni sur sa face opposée d'une couche anti-adhésive, et la seconde partie du rabat est adaptée, avant utilisation, pour être fixée de façon détachable sur la couche anti-adhésive de l'élément d'accrochage.

Ainsi, le rabat muni de ladite troisième partie recouverte d'un élément de masquage permet, à la suite d'une première utilisation dudit rabat, après réouverture de la couche-culotte par déchirure du rabat entre lesdites seconde et troisième parties et après détachement dudit élément de masquage, de refermer la couche-culotte à l'aide dudit rabat, ladite troisième partie débarrassée de l'élément de masquage étant alors fixée par sa couche d'adhésif à ladite autre partie de ladite feuille imperméable de la couche-culotte.

Pour assurer que, lors de la réouverture du dispositif à la suite de la première fermeture, la déchirure du rabat s'effectue à l'endroit voulu entre lesdites seconde et troisième parties du rabat, il est avantageux que ce dernier présente à cet endroit une amorce de déchirure, par exemple deux entailles marginales opposées ou une ligne d'affaiblissement constituée par exemple par une ligne de perforations.

De préférence, ledit élément d'accrochage est fixé par sa couche d'adhésif sur la face interne de la partie arrière de la feuille imperméable et ledit rabat est fixé par la couche d'adhésif de sa première partie sur la face externe de la partie arrière de la feuille imperméable.

Le rabat est avantageusement réalisé de manière qu'avant utilisation sa troisième partie recouverte par l'élément de masquage se trouve située sur la face de la feuille imperméable sur laquelle est fixé ledit élément d'accrochage, entre le bord de l'enveloppe et l'élément d'accrochage, ou la partie de l'élément d'accrochage à laquelle est fixée de façon détachable ladite seconde partie du rabat, tandis que la première partie du rabat est fixée de façon non détachable sur la face opposée de la feuille imperméable.

Pour faciliter, lors de la première utilisation du dispositif de fermeture, le détachement de la seconde partie du rabat de l'élément d'accrochage, il est avantageux que l'extrémité libre de ladite seconde partie du rabat soit repliée sur elle-même. De cette manière, l'extrémité libre de la seconde partie n'adhère pas à l'élément d'accrochage et peut être saisie sans aucune difficulté.

En se référant aux dessins schématiques annexés, on va décrire ci-après plus en détail un mode de réalisation préféré de l'invention, donné à titre d'exemple illustratif et non limitatif ; sur les dessins :

la fig. 1 est une vue en perspective d'une couche-culotte à jeter équipée de deux dispositifs d'attache par adhésif conformes à l'invention ;

la fig. 2 est une coupe longitudinale d'un dispositif d'attache avant sa première utilisation ;

la fig. 3 est une coupe longitudinale du dispositif, lors de la première fermeture ;

la fig. 4 est une coupe longitudinale du dispositif, lors de la seconde fermeture.

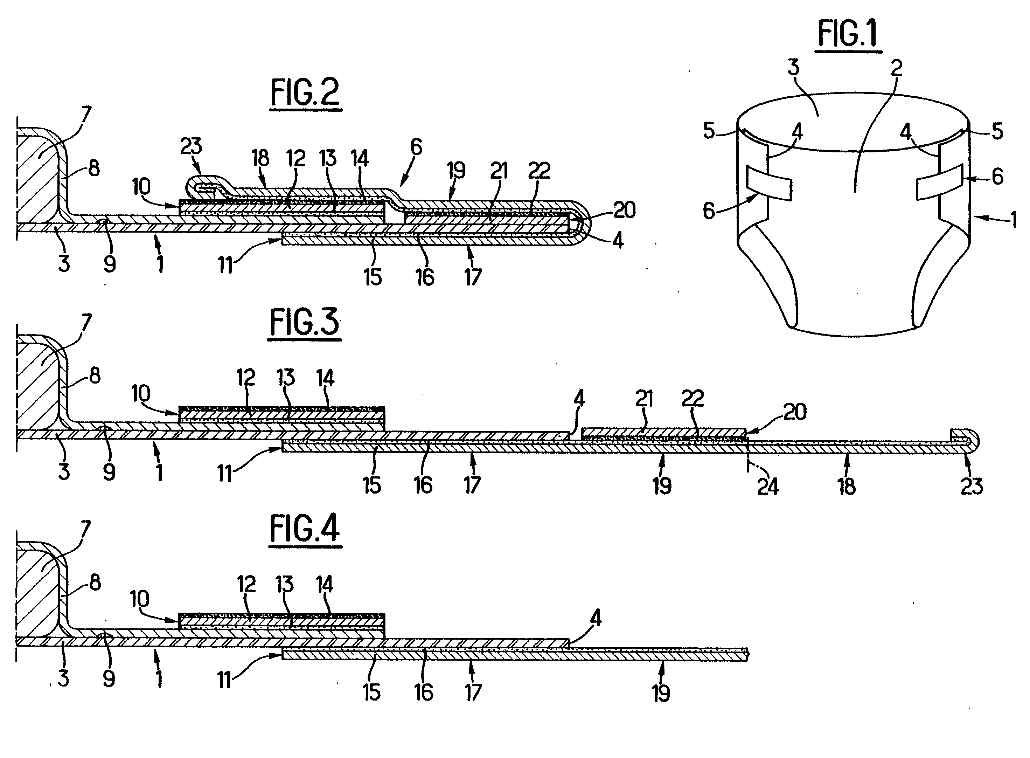

Il convient de noter que sur les fig. 2 à 4, les différents éléments constitutifs ont été illustrés, pour des raisons de clarté du dessin, avec des épaisseurs exagérées.

La couche-culotte à jeter illustrée par la fig. 1 comprend principalement une enveloppe constituée par une mince feuille extérieure 1 souple, imperméable, par exemple en polyéthylène. Cette feuille 1 de forme générale rectangulaire adopte, lorsqu'elle est posée sur l'enfant, la forme visible sur la fig. 1, avec une partie avant 2 et une partie arrière 3 dont les deux bords latéraux opposés 4 recouvrent les bords latéraux opposés 5 de la partie avant 2.

Pour pouvoir fermer la couche-culotte à hauteur de la taille de l'enfant, la partie arrière 3 comporte, sur chaque bord latéral 4, un dispositif d'attache par adhésif 6 coopérant avec la partie avant 2.

Sur la fig. 2 qui représente en coupe la zone d'un bord latéral 4 de la partie arrière 3 de la couche-culotte, avec l'un des dispositifs d'attache 6, on reconnaît un matelas absorbant 7 posé sur la face interne la feuille 1, ainsi qu'une feuille intérieure perméable 8, par exemple un voile de non tissé, recouvrant le matelas 7 ainsi que la face interne de la feuille 1 à laquelle il est fixé par des lignes de colle 9.

Le dispositif d'attache 6 représenté sur la fig. 2 dans la position qu'il occupe avant l'utilisation comprend un élément d'accrochage 10 et un rabat 11.

L'élément d'accrochage 10 est constitué par un matériau 12 en feuille muni sur l'une de ses faces d'une couche d'adhésif 13 et sur sa face opposée d'une couche 14 anti-adhésive. L'élément d'accrochage 10 est fixé par la couche d'adhésif 13 sur le voile 8, à distance du bord latéral 4 de la feuille 1.

Le rabat 11 est constitué par un matériau de support 15 en forme de feuille, muni sur l'une de ses faces d'une couche 16 d'adhésif sensible à la pression. Le rabat 11 présente une largeur égale ou légèrement inférieure à la largeur de l'élément d'accrochage 10, mais une longueur nettement supérieure à celle de l'élément d'accrochage 10.

A l'une de ses extrémités longitudinales, le rabat 11 est fixé par une première partie 17, par la couche d'adhésif 16, sur la face externe de la feuille 1, les parties restantes 18 et 19 du rabat 11 étant rabattues autour du bord latéral 4 de la feuille 1 de façon que la partie 18 vienne se placer sur l'élément d'accrochage 10 et adhère par sa couche d'adhésif 16 sur la couche anti-adhésive 14 de l'élément 10. Sur la partie 19 du rabat 11 située entre le bord latéral 4 de la feuille 1 et l'élément d'accrochage 10, la couche d'adhésif 16 est recouverte par un élément de masquage 20 empêchant la couche d'adhésif 16 d'adhérer ici à la face intérieure de la feuille 1. Cet élément de masquage 20 ayant une largeur égale ou supérieure à la largeur du rabat 11 est constituée par un matériau de support 21 en forme de feuille muni d'une couche 22 anti-adhésive sur sa face tournée vers la couche d'adhésif 16 de la partie 19 du rabat 11.

La partie 18 du rabat 11 qui est appliquée par sa couche d'adhésif 16 sur l'élément d'accrochage 10 n'adhère que modérément à la couche anti-adhésive 14 de ce dernier. Son extrémité libre est repliée sur elle-même en 23 de sorte que cette extrémité libre 23 n'adhère pas à l'élément d'accrochage 10.

Pour fermer la couche-culotte à l'aide du dispositif d'attache 6, on saisit l'extrémité libre repliée 23 de la partie 18 du rabat 11 et on détache la partie 18 de l'élément d'accrochage 10. Après avoir amené dans le prolongement de la partie 17 du rabat 11, non seulement la partie 18 mais également la partie 19 avec l'élément de masquage 20 qui empêche la partie 19 d'adhérer à la feuille 1, on fixe la partie 18 par sa couche d'adhésif 16 sur la face extérieure de la feuille 1 de la partie avant 2 de la couche-culotte, le bord latéral 4 de la partie arrière 3 recouvrant le bord latéral 5 de la partie avant 2. Dans cette position fermée qui est illustrée par la fig. 3, l'élément de masquage 20 empêche la partie 19 du rabat 11 d'adhérer à la feuille 1 de la partie avant 2.

Pour ouvrir de nouveau la couche-culotte sans détruire définitivement le dispositif d'attache, on déchire le rabat 11 en 24, entre la partie 18 du rabat 11 fixée à la partie avant 2 de la couche-culotte, et la partie 19 du rabat 11, recouverte par l'élément de masquage 20. Pour faciliter cette déchirure, le rabat 11 est avantageusement muni en 24 d'une amorce de déchirure, par exemple une ou deux entailles latérales opposées ou une ligne transversale de perforations.

Pour refermer ensuite la couche-culotte, on détache l'élément de masquage 20 de la partie 19 du rabat 11, ce qui ne présente pas de difficulté puisque la couche anti-adhésive 22 de l'élément de masquage 20 se trouve en contact avec la couche d'adhésif 16 de la partie 19 du rabat 11. La couche d'adhésif 16 de la partie 19 du rabat 11 étant ainsi mise à nu, on fixe la partie 19 du rabat 11 dans la position voulue sur la face extérieure de la feuille 1 de la partie avant 2 de la couche-culotte.

Des adhésifs sensibles à la pression pour la couche 16 du rabat 11 et des produits anti-adhésifs, par exemple à base de silicone, pour la couche 14 de l'élément d'accrochage 10 et la couche 22 de l'élément de masquage 20 sont bien connus par l'homme de l'art et leurs compositions n'entrent pas dans le cadre de la présente invention.

Bien entendu, le mode de réalisation décrit ci-dessus et représenté sur le dessin annexé, n'a été donné qu'à titre illustratif et non limitatif et peut recevoir de nombreuses modifications et variantes dans le cadre de l'invention. Ainsi, le rabat 11 pourrait également être fixé de façon non détachable par sa partie 17 sur la face intérieure de l'enveloppe 1. Par ailleurs, au lieu d'être fixé sur la partie arrière 3 de la couche-culotte, le rabat 11 pourrait être fixé sur la partie avant 2. Il convient cependant de noter que le mode de fixation représenté et décrit procure la plus grande simplicité d'utilisation.

L'invention s'applique à tous les types de couches-culottes, notamment avec ou sans élastiques à la taille et/ou à l'entrejambe, avec matelas absorbant de toutes formes s'étendant jusqu'aux bords latéraux de la feuille extérieure ou se terminant à distance de ces bords, avec ou sans échancrures pour les jambes, etc...

**Revendications**

1. Couche-culotte à jeter comprenant une mince feuille extérieure imperméable (1), un matelas absorbant (7) et de préférence une feuille interne perméable (8), deux dispositifs d'attache par adhésif (6) placés sur les bords latéraux de la couche-culotte et destinés à fixer l'une à l'autre les parties avant et arrière (2, 3) de la couche-culotte, chaque dispositif d'attache comprenant : un rabat de fixation (11) qui présente à une extrémité une première partie (17) fixée de façon non détachable par une couche d'adhésif (16) sur la face externe de la partie arrière (3) de ladite feuille imperméable (1) jusqu'au bord latéral de ladite partie arrière (3), ainsi que, à son autre extrémité, une seconde partie (18) qui est destinée au cours de l'utilisation, à être fixée par une couche d'adhésif (16) sensible à la pression sur la face externe de la partie avant (2) de la feuille imperméable (1), ledit rabat (11) comprenant, entre lesdites première et seconde parties (17, 18) une troisième partie (19) qui est munie de la même couche d'adhésif (16) sensible à la pression que lesdites première et seconde parties (17, 18) et qui est recouverte d'un élément de masquage (20) dont la face tournée vers ladite couche d'adhésif (16) dudit rabat (11) est munie d'une couche anti-adhésive (22), caractérisée par le fait que chaque dispositif d'attache comprend en outre un élément d'accrochage (10) fixé de façon non détachable par une couche d'adhésif (13) au voisinage du bord de la face interne de la partie arrière (3) de ladite feuille imperméable (1) et muni sur sa face opposée d'une couche anti-adhésive (14) ; et que la seconde partie (18) du rabat (11) est adaptée, avant utilisation, pour être fixée de façon détachable sur la couche anti-adhésive (14) de l'élément d'accrochage (10).

2. Couche-culotte suivant la revendication 1, caractérisée par le fait que ledit rabat est réalisé de manière qu'avant utilisation, sa troisième partie (19) recouverte par l'élément de masquage (20) se trouve située sur la face interne de la feuille imperméable (1) sur laquelle est fixé ledit élément d'accrochage (10), entre le bord (4) de la feuille imperméable et l'élément d'accrochage, tandis que la première partie (17) du rabat est fixée de façon non détachable sur la face opposée de la feuille imperméable (1).

3. Couche-culotte suivant la revendication 1 ou 2, caractérisée par le fait que ledit rabat présente une amorce de déchirure (24) entre lesdites seconde et troisième parties (18, 19).

4. Couche-culotte suivant l'une quelconque des revendications précédentes, caractérisée par le fait que l'extrémité libre (23) de la seconde partie (18) du rabat (11) est repliée sur elle-même.

**Claims**

1. Disposable integral diaper comprising a thin impervious external sheet (1), an absorbent pad (7) and preferably a permeable inner sheet (8), two devices for attachment by means of adhesive (6) which are placed on the side edges of the integral diaper and are intended to fix the front and rear (2, 3) parts of the integral diaper to each other, each attachment device comprising : a fixing flap (11), which at one end has a first part (17) fixed in an unremovable manner by a layer of adhesive (16) on the outer face of the rear part (3) of the said impervious sheet (1) as far as the side edge of the said rear part (3), and, at its other end, a second part (18) which is intended during use to be fixed by a layer of pressure-sensitive adhesive (16) on the outer face of the front part (2) of the impervious sheet (1), the said flap (11) comprising, between the said first and second parts (17, 18), a third part (19) which is equipped with the same layer of pressure-sensitive adhesive (16) as the said first and second parts (17, 18) and which is covered with a masking member (20) whose side facing the said layer of adhesive (16) of the said flap (11) is equipped with a release layer (22), characterized in that each attachment device additionally comprises a hooking member (10) fixed in an unremovable manner by a layer of adhesive (13) in the vicinity of the edge of the inner face of the rear part (3) of the said impervious sheet (1) and equipped with a release layer (14) on its opposite side ; and in that the second part (18) of the flap (11) is adapted, before use, to be fixed in a removable manner on the release layer (14) on the hooking member (10).

2. Integral diaper according to Claim 1, characterized in that the said flap is made so that, before use, its third part (19) covered by the masking member (20) is situated on the inner side of the impervious sheet (1) on which the said hooking member (10) is fixed, between the edge (4) of the impervious sheet and the hooking member, while the first part (17) of the flap is fixed in an unremovable manner on the opposite side of the impervious sheet (1).

3. Integral diaper according to Claim 1 or 2, characterized in that the said flap has a tear initiator (24) between the said second and third parts (18, 19).

4. Integral diaper according to any one of the preceeding claims, characterized in that the free end (23) of the second part (18) of the flap (11) is folded back onto itself.

**Patentansprüche**

1. Wegwerf-Windelhöschen mit einer undurchlässigen dünnen Außenfolie (1), einer Absorptionseinlage (7) und vorzugsweise einer durchlässigen Innenfolie (8), zwei Klebeverschlußvorrichtungen (6) an den Seitenrändern des Windelhöschens, die dazu bestimmt sind, das Vorderteil und das Rückenteil (2, 3) des Windelhöschens miteinander zu verbinden, wobei jeder Klebeverschluß enthält : Eine Befestigungsklappe (11) mit einem ersten Teil (17) an einem Ende, der auf nicht ablösbare Weise mit einer Klebeschicht (16) an der Außenseite des rückwärtigen Teils (3) der genannten undurchlässigen Folie (1) bis zum Seitenrand des genannten rückwärtigen Teils (3) befestigt ist, sowie, an seinem anderen Ende, einen zweiten Teil (18), der bei Gebrauch mit einer druckempfindlichen Klebeschicht (16) auf die Außenseite des Vorderteils (2) der undurchlässigen Folie (1) befestigt wird, wobei die genannte Klappe (11) zwischen dem genannten ersten und dem genannten zweiten Teil (17, 18) einen dritten Teil (19) enthält, der mit der gleichen auf Druck ansprechenden Klebeschicht (16) versehen ist wie der genannte erste und zweite Teil (17, 18), und der mit einem Deckelement (20) bedeckt ist, dessen der genannten Klebeschicht (16) auf der genannten Klappe (11) zugewandte Seite mit einer sogenannten release Schicht (22) versehen ist, dadurch gekennzeichnet, daß jede Verschlußvorrichtung ferner ein Haftelement (10) enthält, das durch eine Klebeschicht (13) am Rand der Innenseite des rückwärtigen Teils (3) der genannten undurchlässigen Folie (1) in nicht lösbarer Verbindung befestigt ist und auf seiner gegenüberliegenden Seite mit einer sogenannten release Schicht (14) versehen ist, und daß der zweite Teil (18) der Klappe (11) vor Gebrauch so angepaßt ist, daß er in lösbarer Verbindung auf der release Schicht (14) des Haftelements (10) befestigt ist.

2. Windelhöschen gemäß Anspruch 1, dadurch gekennzeichnet, daß die genannte Klappe so ausgeführt ist, daß vor Gebrauch ihr vom Deckelement (20) bedeckter dritter Teil (19) auf die Innenfläche der undurchlässigen Folie (1), auf der das genannte Haftelement (10) befestigt ist, zwischen dem Rand (4) der undurchlässigen Folie und dem Haftelement zu liegen kommt, während der erste Teil (17) der Klappe in nicht lösbarer Weise auf der gegenüberliegenden Seite der undurchlässigen Folie befestigt ist.

3. Windelhöschen gemäß Ansprüchen 1 oder 2, dadurch gekennzeichnet, daß die genannte Klappe eine Sollreißstelle (24) zwischen dem genannten zweiten und dritten Teil (18, 19) enthält.

4. Windelhöschen gemäß einem beliebigen der vorstehenden Ansprüche, dadurch gekennzeichnet, daß das freie Ende (23) des zweite Teils (18) der Klappe (11) umgeschlagen ist.

FIG.1

FIG.2

FIG.3

FIG.4

0 080 152